# EUROPEAN PATENT SPECIFICATION

(11) **EP 2 233 588 B1**
(45) Date of publication and mention of the grant of the patent: **15.05.2013**
(21) Application number: 09003909.0
(22) Date of filing: 18.03.2009
(51) Int. Cl.: C12Q 1/68

(54) **In vitro methods for determining an intracellular free magnesium deficiency of individuals by determining the expression level of a cellular Mg2+ transporter gene**
In-vitro-Verfahren zur Bestimmung eines intrazellulären Mangels an freiem Magnesium in Personen, indem die Expressionsniveau eines zellulären Mg2+-Transportergens bestimmt wird
Procédés in vitro pour déterminer une déficience en magnésium libre intracellulaire d'individus en déterminant le niveau d'expression d'un gène transporteur Mg2+ cellulaire

(43) Date of publication of application: 29.09.2010
(73) Proprietor: Protina Pharmazeutische Gesellschaft mbH, 85737 Ismaning (DE)
(72) Inventor: Kolisek, Martin Dr., 14169 Berlin (DE); Vormann, Jürgen Prof. Dr., 85662 Hohenbrunn (DE)
(74) Representative: Eisenführ, Speiser & Partner

(56) References cited:
- US-A1- 2008 090 297
- GOYTAIN ANGELA ET AL: "Functional characterization of ACDP2 (ancient conserved domain protein), a divalent metal transporter." PHYSIOLOGICAL GENOMICS 11 AUG 2005, vol. 22, no. 3, 11 August 2005 (2005-08-11), pages 382-389, XP002535555 ISSN: 1531-2267
- KOLISEK MARTIN ET AL: "SLC41A1 is a novel mammalian Mg2+ carrier." THE JOURNAL OF BIOLOGICAL CHEMISTRY 6 JUN 2008, vol. 283, no. 23, 6 June 2008 (2008-06-06), pages 16235-16247, XP002535556 ISSN: 0021-9258
- FRANZ KAY B: "A functional biological marker is needed for diagnosing magnesium deficiency." JOURNAL OF THE AMERICAN COLLEGE OF NUTRITION DEC 2004, vol. 23, no. 6, December 2004 (2004-12), pages 738S-41S, XP002535557 ISSN: 0731-5724
- ARNAUD MAURICE J: "Update on the assessment of magnesium status." THE BRITISH JOURNAL OF NUTRITION JUN 2008, vol. 99 Suppl 3, June 2008 (2008-06), pages S24-S36, XP002535558 ISSN: 1475-2662
- SCHMITZ CARSTEN ET AL: "Molecular components of vertebrate Mg2+-homeostasis regulation" MAGNESIUM RESEARCH, JOHN LIBBEY, LONDON, GB, vol. 20, no. 1, 1 March 2007 (2007-03-01), pages 6-18, XP009119389 ISSN: 0953-1424
- ROMANI ET AL: "Regulation of magnesium homeostasis and transport in mammalian cells" ARCHIVES OF BIOCHEMISTRY AND BIOPHYSICS, ACADEMIC PRESS, US, vol. 458, no. 1, 3 February 2007 (2007-02-03), pages 90-102, XP005866967 ISSN: 0003-9861

### SEQUENCE LISTING

<110> Protina Pharmazeutische Gesellschaft mbH
<120> *In vitro* methods for determining an intracellular free magnesium deficiency of individuals by
   determining the expression rate of a cellular Mg²⁺ transporter gene
<160> 22
<210> 1
   <211> 22
   <212> DNA
   <213> artificial sequence
<220>
   <223> Primer
<400> 1
   gattctcctg tacatcgcag ac 22
<210> 2
   <211> 20
   <212> DNA
   <213> artificial sequence
<220>
   <223> Primer
<400> 2
   cccctatgag ccagagaaca 20
<210> 3
   <211> 20
   <212> DNA
   <213> artificial sequence
<220>
   <223> Primer
<400> 3
   atcaagctgc agccaaaagt 20
<210> 4
   <211> 20
   <212> DNA
   <213> artificial sequence
<220>
   <223> Primer
<400> 4
   gctagcaaaa agggcacaag 20
<210> 5
   <211> 24
   <212> DNA
   <213> artificial sequence
<220>
   <223> Primer
<400> 5
   cacaaagata gtcggtatct gacg 24
<210> 6
   <211> 19
   <212> DNA
   <213> artificial sequence
<220>
   <223> Primer
<400> 6
   gaccatggcc aggatgatt 19
<210> 7
   <211> 20
   <212> DNA
   <213> artificial sequence
<220>
   <223> Primer
<400> 7
   tgcaggtgat cttcatttcg 20
<210> 8
   <211> 20
   <212> DNA
   <213> artificial sequence
<220>
   <223> Primer
<400> 8
   gcagtgagca cagcaggtag 20
<210> 9
   <211> 20
   <212> DNA
   <213> artificial sequence
<220>
   <223> Primer
<400> 9
   gggattgctt ttggctgtta 20
<210> 10
   <211> 20
   <212> DNA
   <213> artificial sequence
<220>
   <223> Primer
<400> 10
   tatgggcata tggtggtcct 20
<210> 11
   <211> 20
   <212> DNA
   <213> artificial sequence
<220>
   <223> Primer
<400> 11
   ggatctctct gccctgactg 20
<210> 12
   <211> 20
   <212> DNA
   <213> artificial sequence
<220>
   <223> Primer
<400> 12
   ttctctccag cgatctccat 20
<210> 13
   <211> 20
   <212> DNA
   <213> artificial sequence
<220>
   <223> Primer
<400> 13
   tgggaaggct gaatatgagg 20
<210> 14
   <211> 20
   <212> DNA
   <213> artificial sequence
<220>
   <223> Primer
<400> 14
   tcgctgtcat ccattgtcat 20
<210> 15
   <211> 20
   <212> DNA
   <213> artificial sequence
<220>
   <223> Primer
<400> 15
   aacaacccgt ccagtcagag 20
<210> 16
   <211> 20
   <212> DNA
   <213> artificial sequence
<220>
   <223> Primer
<400> 16
   gtagtagatg gccccgaaca 20
<210> 17
   <211> 20
   <212> DNA
   <213> artificial sequence
<220>
   <223> Primer
<400> 17
   atggaatgga gttccagacg 20
<210> 18
   <211> 20
   <212> DNA
   <213> artificial sequence
<220>
   <223> Primer
<400> 18
   tcattagctt gcctgcacac 20
<210> 19
   <211> 20
   <212> DNA
   <213> artificial sequence
<220>
   <223> Primer
<400> 19
   ggacttcgag caagagatgg 20
<210> 20
   <211> 20
   <212> DNA
   <213> artificial sequence
<220>
   <223> Primer
<400> 20
   agcactgtgt tggcgtacag 20
<210> 21
   <211> 20
   <212> DNA
   <213> artificial sequence
<220>
   <223> Primer
<400> 21
   gccctacaac tccatcctca 20
<210> 22
   <211 > 20
   <212> DNA
   <213> artificial sequence
<220>
   <223> Primer
<400> 22
   gtcaacattc agggctccat 20

## Description

### Background

Magnesium is an essential microelement for over four hundred fundamental molecular processes in the cell and also for various physiological processes at the tissue, organ and organism levels. In order to maintain the various functions of magnesium, both the extracellular and intracellular magnesium concentrations are regulated by complex control mechanisms. Principally, the cellular magnesium homeostasis is maintained by different transport systems which carry magnesium ions from the exterior into the cell or vice versa.

Magnesium deficiency at both the cell and organism levels has been demonstrated to be involved in the development and/or symptoms of various diseases, including so-called "Western" diseases. These are in particular: metabolic syndrome involving insulin resistance, diabetes mellitus type 2 (DM2; approximately 30% of the patients are magnesium-insufficient), obesity, essential hypertension, atherosclerosis and cardiomyopathy; acute heart infarction; brain-stroke, psychoses and various psychiatric diseases and neurodegenerative diseases.

Currently, the clinical testing for magnesium insufficiency is limited to the determination of blood plasma total magnesium (BPM; sum of bound and ionised magnesium) or a similar determination in liquor. However, the determination of the total magnesium content in a cell is not very informative since the major part of the magnesium is bound but only that part which is freely available (as magnesium ions) affects the activity of biochemical systems. The following techniques of the prior art principally enable the determination of Mg²⁺ concentration in cells: (1) fast filter mag-fura 2 spectrometry (mag-fura 2 FFS; ratiometric fluorescence measurement), (2) NMR-based ³¹P-NMR spectroscopy and (3) Mg-specific ion-selective electrodes (ISE; electrophysiological technique). The disadvantages of these techniques are their high running costs and their technical complexity which demands specially trained personnel to evaluate the obtained data. Consequently, they are mostly used for research purposes only and a routine clinical test for the evaluation of free intracellular Mg²⁺ is presently not available.

Therefore, the main object of the present invention is to provide a relatively simple and fast method for evaluating the intracellular free magnesium status of an individual, in particular a human individual suspected to suffer from an intracellular magnesium deficiency. Also disclosed are methods for assessing the bioavailability of magnesium compounds in a mammal, in particular a human, and methods for identifying modulators of the intracellular free magnesium concentration.

These objects are achieved by the methods disclosed herein which involve the determination of the expression level of a cellular Mg²⁺ transporter gene.

### Description of the Invention

The method according to the invention for free magnesium status, in determining the presence or absence of an intracellular free magnesium deficiency caused by diabetes mellitus type 2 in a human individual generally comprises determining the expression level of at least one cellular Mg²⁺ transporter gene showing a direct correlation between its expression level and the intracellular free magnesium ion concentration [Mg²⁺]ᵢ in a cell sample of said individual and comparing the obtained expression level with a reference value, wherein an increase of the expression level relative to the reference value indicates that the individual suffers from an intracellular free magnesium deficiency, and wherein the at least one cellular Mg²⁺ transporter gene is CNNM2*.*

More specifically, this method comprises the following steps:
a) performing quantitative PCR on the mRNA of a cellular Mg²⁺ transporter gene showing a direct correlation between its expression level and the intracellular free magnesium ion concentration [Mg²⁺]ᵢ in a nucleated blood cell sample, preferably a leucocyte sample, of the individual;
b) comparing the value obtained in step a) with a reference value obtained from a nucleated blood cell sample, preferably a leucocyte sample, of a healthy human showing no intracellular free magnesium deficiency,
   wherein an increase in the expression level of the cellular Mg²⁺ transporter gene relative to the reference value indicates that the individual suffers from an intracellular free magnesium deficiency.

Preferably, the increase in the expression level of the cellular Mg²⁺ transporter gene relative to the reference value indicating an intracellular free magnesium deficiency is at least 2-fold.

Several genes encoding for Mg²⁺ transporters and/or homeostatic factors have been identified recently in animals (compare references 1-10 below; summarised in Tab. 1) and their expression has been demonstrated to be related to dietary magnesium intake (reflected in intracellular Mg²⁺ concentration) in mice (Goytain and Quamme; 2005-2007; kidney cortex cells) except for gene *N33,* which was identified in a Mg²⁺ starvation study in Caco-2 cells (unpublished results of the inventors).

**Table 1**

| Gene | Protein | Protein Family | Confirmed / Putative Mg²⁺ transporter | Mg²⁺ -regulated expression | Reference | NCBI ID numbers of the human homologues |
|---|---|---|---|---|---|---|
| *SLC41A1* | SLC41A1 | SLC41 | Confirmed / carrier | Yes / mouse model | 1, 2, 3 | 254428 |
| *SLC41A2* | SLC41A2 | SLC41 | Confirmed / carrier | Yes / mouse model | 4 | 84102 |
| *SLC41A3* | SLC41A3 | SLC41 | Putative | Yes / mouse model | No record | 54946 |
| *CNNM2* | CNNM2 (ACDP2) | Cyclin / ACDP | Putative | Yes / mouse model | 5, 6 | 54805 |
| *MagT1* | MagT1 | MagT1 | Confirmed | Yes / mouse model | 9 | 84061 |
| *TRPM6* | TRPM6 | TRPM | Confirmed / channel | Yes / mouse model | 8 | 140803 |
| *TRPM7* | TRPM7 | TRPM | Confirmed / channel | Yes / mouse model | 7 | 54822 |
| *NIPA1* | NIPA1 | NIPA1 | Putative | Yes / mouse model | 10 | 123606 |
| *N33* | N33 (TUSC3) | N33 (TUSC) | Putative | Yes / mouse model | No record | 7991 |

The present inventors were able to show that homologues of these 9 genes are expressed in human leucocytes. Additional experiments with various cultivated cells demonstrated that the expression of some of said human genes was considerably increased if the cells were incubated in a culture medium free of magnesium. This indicates that on the cellular level there is a tendency to compensate the cellular magnesium deficit caused by the magnesium-free medium by an increased production of some Mg²⁺ transporters.

These observations lead to the assumption that the expression of such Mg²⁺ transporter genes might be used as a marker associated with clinical intracellular Mg²⁺-insufficiency and an extensive series of experiments were conducted in order to develop a test system suitable to confirm or invalidate this hypothesis. Surprisingly, the present inventors were finally able to identify several human genes which showed a direct correlation between the expression level of said genes on the transcriptional level and the intracellular Mg²⁺ concentration as determined by conventional methods. The term "direct correlation" as used herein means that said correlation involves a direct dependency or a causal relationship between the correlated features, i.e. expression level and intracellular Mg²⁺ concentration.

The expression level of the gene candidates was determined by quantitative real-time PCR, a standard analytical technique available commonly in mid- to large-sized hospitals nowadays. For this purpose, specific primers were designed against known human sequences of the genes coding for Mg²⁺ transporters as listed in Table 1. In this procedure, commercially available software programs were used to minimize the risk of hairpin structure formation and primer dimerisation. Table 2 shows a compilation of such optimized primer sequences which were used for amplification of the respective mRNAs or relevant portions thereof. Principally, however, it is possible to construct and use other suitable primers as well.

Preferably, freshly isolated leucocytes are used as sample cells in the test method according to the invention for evaluation of the intracellular free magnesium status. In contrast to this, conventionally either erythrocytes or myocytes from muscle biopsies have been used in tests for determining the intracellular free magnesium ion concentration in cells. The use of leucocytes as opposed to myocytes is advantageous since it involves an easy, safe, clinically standardised and minimally invasive method of collecting sample material (blood sampling). Erythrocytes are not suited for use in the present method since they do not contain nuclei and consequently regulations on the transcription level are not possible.

It has been established that patients with Diabetes mellitis Typ 2 (DM2; non insulin-dependent diabetes) have an increased demand for magnesium. Extensive epidemiological studies and intervention studies provide ample evidence that DM2 is linked with an impaired magnesium homeostasis. Therefore, DM2 was chosen as a model for the condition of intracellular free magnesium deficiency and leucocyte samples from DM2 patients were used to identify a number of Mg²⁺ transporter genes as suitable marker for use in the test methods of the present invention. However, the present invention is generally applicable to any condition of intracellular free magnesium deficiency caused by a disease or disorder, in particular selected from the group consisting of diabetes mellitus type 2, metabolic syndrome involving insulin resistance, obesity, essential hypertension, atherosclerosis, cardiomyopathy, acute heart infarction, brain-stroke, psychoses and other psychatric diseases and neurodegenerative diseases. Preferably, the term "intracellular free magnesium deficiency" as used herein corresponds to an intracellular free magnesium ion concentration [Mg²⁺]ᵢ of less than 0.5 mmol/l.

In the experiments outlined in detail in Example 1 below, the expression levels of the human homologues of all nine genes of Tab. 1 were analysed in leucocyte samples from 56 DM2 patients and reference samples from 5 healthy individuals. These experiments demonstrated that the genes *CNNM2, SLC41A1, SLC41A2* and *SLC41A3* were significantly over-expressed in a large subpopulation of DM2 patients, whereas the genes *TRPM6, TRPM7, MagT1, NIPA1* and *N33* showed no difference with respect to the reference group. This result was surprising, in particular for gene *TRPM6,* which had been expected to be markedly over-expressed as well, based on the results in the mouse model. The threshold value for a significant over-expression was a GED value (Gene Expression's *C_{T}* Difference) of 2 or above, i.e. an at least two-fold increased expression compared with a reference value.

Importantly, these patients had normal plasma total magnesium concentration [Mg]_{T} (a commonly used clinical parameter for hypomagnesemia) but a reduced intracellular ionised magnesium concentration [Mg²⁺]ᵢ when compared with the values measured in DM2 samples in which an over-expression of marker-genes had not been detected.

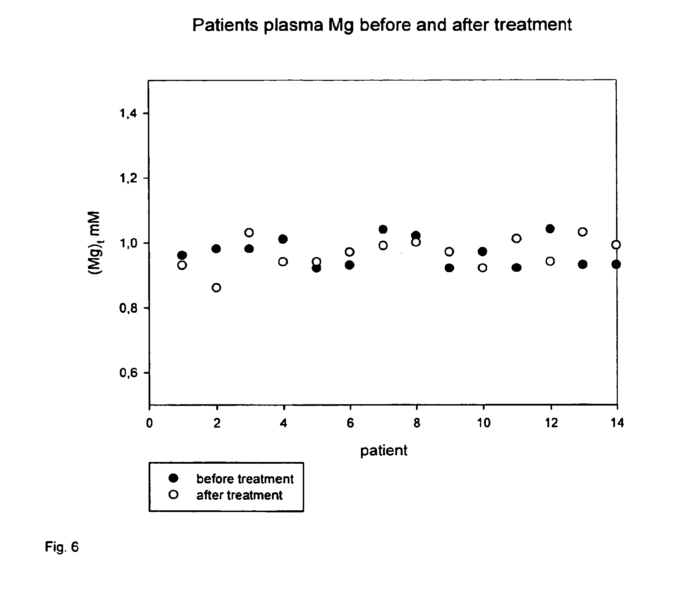

The elevated expression of these marker genes can be suppressed by supplementation of Magnesium-Diasporal^{®} 300 within 30 days of its administration (Example 2). The expression levels of the studied genes in all samples were normalised compared with the expression levels of these genes in the reference samples. Magnesium supplementation had no effect on the concentration of plasma total magnesium values determined before and after the trial (Fig. 6). [Mg²⁺]ᵢ on the other hand was significantly increased in all analysed samples (n=7), demonstrating that an increase of [Mg²⁺]ᵢ actually leads to "normal" expression of the genes of interest.

Further experiments with samples from a control group of healthy donors which regularly took multi-mineral supplements including magnesium showed a normal expression of the *CNNM2, SLC41A1* and *SLC41A3* genes, whereas *SLC41A2* was also over-expressed in these healthy individuals (Example 3). This surprising observation might be connected to the multi-mineral supplementation of the probands in the control group. Its molecular background can however only be speculated upon at present time. Since the expression of *SLC41A2* appears to respond better in a negative manner to factors other than changes of Mg²⁺ concentration in leucocytes, most likely this gene is less suited for use as a marker gene in the methods disclosed herein.

Summarizing, it was possible to identify several human Mg²⁺ transporter genes, in particular *CNNM2, SLC41A1* and *SLC41A3,* which show a direct correlation/dependency between the expression level of said genes on the transcriptional level and the intracellular Mg²⁺ concentration as determined by conventional methods and which can be used as transcriptomic markers for the determination of patient magnesium insufficiency in routine clinical praxis.

However, it will be recognized by a person skilled in the art that further Mg²⁺ transporter genes showing the same properties likely will exist and can be identified with the same or similar techniques as disclosed herein. In particular, the skilled artisan will be able to screen candidate genes which have been identified as Mg²⁺ transporter genes in humans or other mammals for a putative correlation between their expression level and the intracellular free magnesium ion level in a similar manner as disclosed in Example 1 below and to conduct control experiments such as outlined in Examples 2 and 3 below to exclude candidate genes which respond strongly to factors other than changes of intracellular Mg²⁺ concentration.

Thus, the methods disclosed herein are not limited to the specific marker genes as exemplified above and any Mg²⁺ transporter genes showing a direct correlation/dependency between the expression level of said genes on the transcriptional level and the intracellular Mg²⁺ concentration are principally suitable.

Preferably, the Mg²⁺ transporter marker genes suitable for use in the disclosed methods do not include *TRPM6, TRPM7, MagT1, NIPA1, N33,* and/or *SLC41A2.* The marker gene used according to the present invention is *CNNM2.*

The experiments presented in the present specification have been conducted with human test subjects and led to the identification of human marker genes. Consequently, the *in vitro* methods of the invention for determining the presence or absence of an intracellular free magnesium deficiency in an individual relate to methods for evaluating the intracellular free magnesium status of human individuals. However, the skilled artisan will recognize that similar methods may be applied to other mammals where homologues of the above marker genes are expressed.

"Evaluating the intracellular free magnesium status of an individual" as used herein generally means the determination of any decrease or increase of the intracellular Mg²⁺ levels relative to reference values as determined for individuals considered to be normal with respect to said intracellular Mg²⁺ levels. In particular, this term comprises the determination of the presence or absence of an intracellular free magnesium deficiency in an individual. "Normal" preferably means an intracellular free magnesium ion concentration of 0.5 mmol/l or more.

Important benefits of the present invention include a high level of standardisation of the proposed technique resulting in high reproducibility, high through-put, reduced time consumption and low material costs.

Moreover, the approach of the present invention is unique in that it utilizes the fact that the GED values as determined for each marker gene, reflect the individual physiology of the tested person and can be interpreted independently of [Mg²]ᵢ values. This rules out the necessity to conduct analytical determination of [Mg²⁺]ᵢ with one of the above-mentioned analytical techniques and the correlation of the patient's [Mg²⁺]ᵢ with threshold values that are preset artificially (based on statistical evaluation of patient trials) and represents a major progress over the prior art.

The successful identification of such marker genes does not only provide the basis for an improved method of evaluating the intracellular free magnesium status of an individual suspected to suffer from an intracellular free magnesium deficiency as outlined above but also for additional related applications such as novel methods for assessing the bioavailability of magnesium compounds in a mammal and novel methods for identifying modulators of the intracellular free magnesium concentration in a cell.

Thus, a further aspect disclosed herein relates to an *in vitro* method for assessing the bioavailability of magnesium compounds in a mammal by determining the expression level of a cellular Mg²⁺ transporter gene showing a direct correlation between its expression level and the intracellular free magnesium ion concentration [Mg²⁺]ᵢ in a cell, comprising
a) contacting a cell, having an intracellular magnesium deficiency and expressing the cellular Mg²⁺ transporter gene, with the magnesium compound, and
b) determining the expression level of the cellular Mg²⁺ transporter gene and comparing the value obtained with a reference value, with an decrease of said expression level following contact with said magnesium compound indicating bioavailability of said magnesium compound, wherein the cellullar Mg²⁺ transporter gene is CNNM2*.*

Preferably, in this method the amount of decrease of said expression level correlates directly with the degree of bioavailability of said magnesium compound.

In a specific embodiment of this method, the cell having an intracellular magnesium deficiency and expressing the cellular Mg²⁺ transporter gene is selected from cells derived of a culture of magnesium-depleted cells and cell samples derived of a mammal, in particular a human, suffering from an intracellular magnesium deficiency.

In another specific embodiment, the cell having an intracellular magnesium deficiency and expressing the cellular Mg²⁺ transporter gene is a nucleated blood cell, in particular a leucocyte.

The above method is not limited to these specific embodiments and further embodiments and modifications of said method will be readily recognized by the skilled artisan.

A still further aspect disclosed herein relates to an *in vitro* method of identifying a compound that is capable of modulating the intracellular free magnesium ion concentration [Mg²⁺]ᵢ, comprising
a) providing a cell expressing a cellular Mg²⁺ transporter gene showing a direct correlation between its expression level and the intracellular free magnesium ion concentration [Mg²⁺]ᵢ and
b) contacting the cell with a candidate compound, and
c) determining the expression level of the cellular Mg²⁺ transporter gene,
wherein an increase or decrease of said expression level following contact with said candidate compound indicates a modulator of intracellular [Mg²⁺]ᵢ, wherein the cellular Mg²⁺ transporter gene is *CNNM2.*

In a specific embodiment of this method, the cell expressing the cellular Mg²⁺ transporter gene and being contacted with the candidate compound is a cultivated cell from a cell culture contacted with said candidate compound.

In another specific embodiment, the cell expressing the cellular Mg²⁺ transporter gene and being contacted with the candidate compound is a cell sample from a mammal which had been administered said candidate compound.

In a specific embodiment of this method, the cell expressing the cellular Mg²⁺ transporter gene is a human cell. Preferably, the cell expressing the cellular Mg²⁺ transporter gene is a nucleated blood cell, in particular a leucocyte.

The above method is not limited to these specific embodiments and further embodiments and modifications of said method will be readily recognized by the skilled artisan.

A further aspect disclosed herein relates to kits for performing the methods discloses herein. For example, such a kit may comprise reagents for performing quantitative PCR of one or more magnesium transporter marker genes, in particular *CNNM2 (ACDP2), SLC41A1* and *SLC41A3,* and optionally of one or more control genes, e.g. house keeping genes such as *ACTB* or *TUBA1B* in Table 2 below, and optionally further reagents, buffers etc.

Specifically, an exemplary kit may comprise
1.) Reference cDNA* SLC41A1 .... 1 vial a,- 50 reactions
2.) Reference cDNA* SLC41A3 .... 1 vial a,- 50 reactions
3.) Reference cDNA* CNNM2 .......1 vial a,- 50 reactions
4.) Primers set⁺ (forv.; rev.) SLC41A1 .... 1 vial a,- 50 reactions
5.) Primers set⁺ (forv.; rev.) SLC41A3 .... 1 vial a,- 50 reactions
6.) Primers set⁺ (forv.; rev.) CNNM2 .......1 vial a,- 50 reactions

*Reference cDNA vials will contain synthetic cDNA fragments with length of typically app. 300 bases. The sequence of the fragments will be identical with primer target sequences of, e.g., *SLC41A1,* or *SLC41A3,* or *CNNM2* (*ACDP2*). The copy number of template cDNA-fragments will preferably correspond to the template copy numbers which were empirically determined with reference control samples in quantitative PCR experiments conducted before.

### Brief Description of the Figures

Fig. 1 shows the GED expression levels of four over-expressing genes from Tab. 1.
Fig. 2 shows a direct comparison of the expression of all nine genes listed in Tab. 1 in reference leucocytes and DM2 leucocytes (PCR products after gelelectrophoresis).
Fig. 3 shows a plot of the individual values of plasma [Mg]_{T} and [Mg²⁺]ᵢ data acquired for DM2-group patients.
Fig. 4 shows correlation analyses between the GED values for each of said four genes and the corresponding sets of [Mg²]ᵢ (upper panels) and plasma [Mg]_{T} (lower panels), respectively: (4A) *CNNM2,* (4B) *SLC41A1,* (4C) *SLC41A2,* (4D) *SLC41A3.*
Fig. 5 shows the GED values for each of the same genes in DM2 patients before and after supplementation with 300 mg Mg²⁺: (5A) *CNNM2,* (5B) *SLC41A2,* (5C) *SLC41A1,* and (5D) *SLC41A3.*
Fig. 6 shows the plasma total magnesium concentration [Mg]_{T} of DM2 patients before and after supplementation.
Fig. 7 shows the GED values of *CNNM2, SLC41A1, SLC41A2* and *SLC41A3* in healthy donors supplemented with multi-mineral preparations.

The following non-limiting examples illustrate the present invention in more detail.

### EXAMPLE 1

### Analysis of the expression levels of Mg²⁺ transporter genes in leucocyte samples from DM2 patients and reference samples from healthy individuals

It is established that patients with Diabetes mellitis Typ 2 (DM2; non insulin-dependent diabetes) have an increased demand for magnesium. Extensive epidemiological studies and intervention studies provide ample evidence that DM2 is linked with an impaired magnesium homeostasis. Therefore, DM2 patients and healthy individuals were selected as test subjects from which the leucocyte samples were obtained.

Group DM2: The test subjects (56) were all suffering from *diabetes mellitus* type 2 (DM2). All were treated with anti-diabetics; none of them was taking insulin or combined (insulin/anti-diabetics) therapy. The patients were 40 to 80 years old.

Control Group: The test subjects (17) were healthy blood donors. They were 20 to 55 years old.

Reference Group: The test subjects (5) were healthy individuals with no family history of DM2; their age ranged from 25 to 46 years.

The gene amplification efficiencies (GEDs) that were acquired for the each patient / proband of the DM2 group and in the control group were always evaluated against GEDs obtained for the subjects in the reference group.

### Step 1) Isolation of leucocytes

Blood (25-40 ml) was sampled by using the Vacutainer^{®} system and collected into 15 ml heparin-coated test-tubes (lithium heparin 170 I.U.; both BD, Plymouth, UK). After being sampled, the blood samples were transferred into Leucosep^{®} 50 ml tubes pre-filled with Ficoll-Paque™ Plus (Greiner Bio-One; Frickenhausen, Germany). Leucocyte fractions were prepared according to the manufacturer's protocol.

### Step 2) RNA isolation

Total RNA was purified from the leucocyte fractions (from step 1) by the Nucleospin^{®} RNAII mini-kit (Macherey & Nagel, Düren, Germany) according to the manufacturer's protocol. Purified RNA was subsequently analysed by spectrometry (260/280 nm) and its integrity was checked by an Agilent 2100 Bioanalyzer / RNA 6000 Nano LabChip (Agilent; Santa Clara, CA, USA). Only RNA samples with a RIN (RNA integrity number) 6 and higher were taken for further processing.

### Step 3) cDNA synthesis

Reverse transcription of mRNAs was performed by using iScript cDNA synthesis Kit (BioRad; Hercules, CA, USA). The final reaction mixture (20 µl) contained 4 µl of the ready to use reaction mixture (with supplied random hexamers and oligo(dT) primers), 1 µl of reverse transcriptase, sample (a variable volume containing 100 ng of RNA) and molecular biology grade RNAse-free water (a variable volume dependent on the sample volume). To synthesise cDNA, a three-step protocol (25 °C / 5 mins; 42 °C / 30 mins and 85 °C / 5 mins) was used in a Cyclone 25 thermal cycler (PEQLAB Biotechnologie; Erlangen, Germany).

Annotation: RNA transport and storage were performed at -70 °C to -80 °C. When manipulated in the laboratory, sample aliquots were stored on ice and thawing-freezing cycles were reduced to a maximum of one.

### Step 4) Primer design for quantitative PCR

Primers were designed by the software Primer 3. The risks of the hairpin structure formation and of primer dimerisation were analysed by using OligoAnalyzer software. Basic information about the primers are summarised in Table 1. The length of the primers is from 19-mer to 24-mer). GC content is from 45% to 60% and the length of the expected PCR product ranges from 141 bp to 234 bp. As normalisation standards for gene expression analysis, two commonly used housekeeping genes (ACTB and TUBA1B) were selected. Oligonucleotide synthesis was performed by MWG Biotech AG (Ebersberg, Germany). Lyophilised primers were solubilised in DEPC water to give a final concentration of 100 pm.µl⁻¹.

**Table 2**

| Gene | Accession No. | Primer 5'→ 3' | Primer length | SEQ-ID-No. | Amplicon size |
|---|---|---|---|---|---|
| SLC41A1 | NM_173854 | Fw: gattctcctgtacatcgcagac | 22 | 1 | 155 |
| | | Rev: cccctatqaqccaqaqaaca | 20 | 2 | |
| SLC41A2 | NM_032148 | Fw: atcaagctgcagccaaaagt | 20 | 3 | 204 |
| | | Rev: gctagcaaaaagggcacaag | 20 | 4 | |
| SLC41A3 | NM_001008485 | Fw: cacaaagatagtcggtatctgacg | 24 | 5 | 144 |
| | | Rev: gaccatggccaggatgatt | 19 | 6 | |
| CNNM2 | NM_017649 | Fw: tgcaggtgatcttcatttcg | 20 | 7 | 189 |
| | | Rev: gcagtgagcacagcaggtag | 20 | 8 | |
| MAGT1 | NM_032121 | Fw: gggattgcttttggctgtta | 20 | 9 | 164 |
| | | Rev: tatgggcatatggtggtcct | 20 | 10 | |
| TRPM6 | NM_017662 | Fw: ggatctctctgccctgactg | 20 | 11 | 177 |
| | | Rev: ttctcccagcgatctccat | 20 | 12 | |
| TRPM7 | NM_017672 | Fw: tgggaaggctgaatatgagg | 20 | 13 | 157 |
| | | Rev: tcgctgtcatccattgtcat | 20 | 14 | |
| NIPA1 | NM_144599 | Fw: aacaacccgtccagtcagag | 20 | 15 | 141 |
| | | Rev: gtagtagatggccccgaaca | 20 | 16 | |
| N33 | NM_006765 | Fw: atggaatggagttccagacg | 20 | 17 | 153 |
| | | Rev: tcattagcttgcctgcacac | | 18 | |
| ACTB (house-keeper) | NM_001101 | Fw: ggacttcgagcaagagatgg | 20 | 19 | 234 |
| | | Rev: agcactgtgttggcgtacag | 20 | 20 | |
| TUBA1B (house-keeper) | NM_006082 | Fw: gccctacaactccatcctca | 20 | 21 | 205 |
| | | Rev: gtcaacattcagggctccat | 20 | 22 | |

### Step 5) Quantitative PCR

Primer specificity has been tested by conventional PCR, prior to quantitative PCR. PCR products were electroseparated on 2% agarose gel, stained with ethidium bromide and visualised by UV light (Bio-imager; Alphalnnotech; San Leonardo, CA, USA). If necessary, the annealing temperatures of the primers were tested by temperature gradient PCR (Primus 96 advanced, PEQLAB).

Quantitative PCR was performed by using SYBR^{®}Green based mix (iQ SYBR Green Supermix; BioRad) containing SYBR Green I dye, hot start i*Taq* DNA polymerase, buffer and dNTPs. The final reaction volume was 25 µl: iQ SYBR Green Supermix (13 µl), DEPC water (7.5 µl), forward primer (0.5 µl), reverse primer (0.5 µl) and template DNA (25 µl). Amplifications (triplicate for each sample) were conducted in 96-well plates by iCycler (BioRad).

Three optimised real-time PCR programs were used depending on previous melting-point analyses for each respective gene. For *SLC41A1, A2, A3,* and *MAGT1, N33* and *CNNM2,* a two-step protocol was applied [(95 °C/30"-58 °C/120") x 35]. The TRPM6/7fragment was amplified by using a three-step protocol: [(95 °C/30"-58 °C/120"-78 °C/10") x 35], and the NIPA1 fragment, by using a three-step protocol: [(95 °C/30"-58 °C/120"-80 °C/10") x 35].

Data were acquired with iQ5 signal detection software (BioRad) and analysed according to the GED method (Gene Expression's *C_{T}* Difference) of Schefe et al. (2006) except that software FC-KW.01 (Katarina Wolf, FU Berlin) was used instead of the IinReg PCR software used by Schefe et al. (2006), because of its improved algorithm which enables to process sample singlets instead of sample triplets, but principally each of these programs or a similar data analysis program of the prior art is suitable for this step.

Amplified products were again size-analysed on a 2% agarose gel.

### Step 6) Plasma total [Mg] AAS analysis

Assessment of the plasma total magnesium concentration was performed for each sample by flame atomic absorption spectrometry (AAS; iCE 3300, Thermo Scientific). Total magnesium concentrations were calculated by SOLAAR AAS software (Thermo Scientific).

### Step 7) Intracellular ionised [Mg²⁺] FFS ratiometric analyse

Collected leucocytes were resuspended, washed twice with PBS (cells were pelleted at 1700 rpm; 5 mins) and loaded with mag-fura 2 (ratiometric Mg²⁺-sensitive fluorochrome; Invitrogen) in divalent-cation-free HBS (Hank's Balanced Solution) in the presence of Pluronic F-127 (Invitrogen) following the same loading protocol as described in Kolisek et al. (2008). Measurements of [Mg²] were performed by using an LS55 spectrophotometer equipped with a fast filter accessory and biokinetic head (all purchased from Perkin Elmer). The duration of each measurement was set at 100 s. Technical details and calibration of the measurements were as described by Kolisek et al. (2008), Schweigel et al. (2006) and Kolisek et al. (2003). Acquired data were processed by using the Grynkiewicz equation (Grynkiewicz et al., 1985) and the final intracellular [Mg²⁺] was calculated (FL WinLab, Perkin Elmer).

Summary of the results: Genes *CNNM2, SLC41A1, SLC41A2* and *SLC41A3* are significantly over-expressed in leucocytes of the sub-population of DM2 patients; the increase of their expression correlates with a decrease of [Mg²⁺]ᵢ in these cells but not plasma [Mg]_{T}.

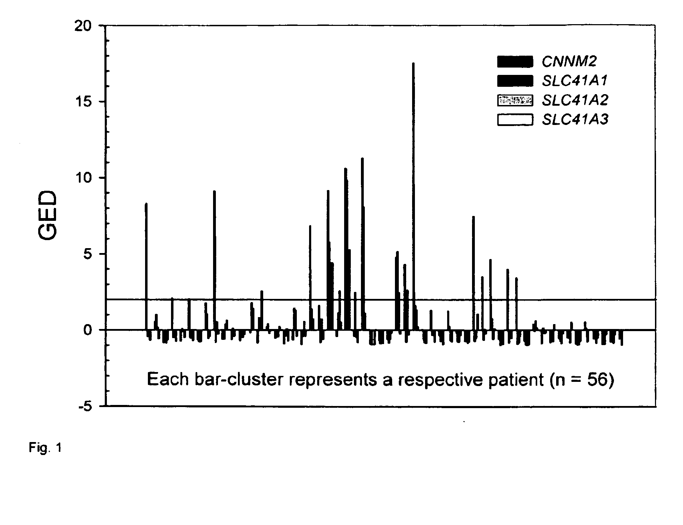

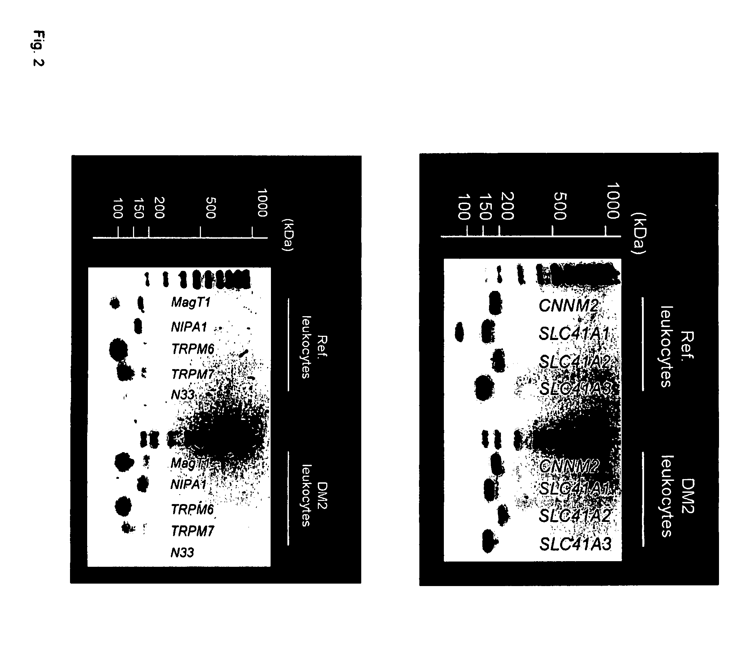

Detailed results: The expression levels of all nine genes (Tab. 1) have been analysed and the levels of four over-expressing genes have been plotted together (Fig. 1; GED threshold/red line = 2). Tests have revealed that, among 56 probands, 16 (29% of all patients, GED threshold = 2) had a significantly over-expressed (up to 17.5 times) gene *CNNM2.* 5 of these patients (9% of all patients, GED threshold = 2) had a significantly over-expressed (up to 8.1 times) gene *SLC41A1.* In the DM2 group of patients, we also identified 5 patients (9% of all patients, GED threshold = 2) over-expressing gene SLC41A2 (up to 2.6 times) and three patients (5% of all patients, GED threshold = 2) over-expressing gene SLC41A3 (up to 5.3 times). GED values were normalised to the expression values obtained for these genes in the reference group of probands (n = 5). Although genes: *TRPM6, TRPM7, MagT1, NIPA1* and *N33* were also expressed in isolated leucocytes (Fig. 2), they were not seen to be differentially expressed in the DM2 group and the reference group (real-time PCR analyse). Therefore, they were excluded from further study (Fig. 2, sizes of the PCR products are denoted in Tab. 2). Among the DM2 patients, the over-expression of *CNNM2* was the most significant. As seen in Figure 1 and Table 3, no correlation existed among the over-expression levels of the respective genes *(CNNM2, SLC41A1, SLC41A2* and *SLC41A3*), nor was any common over-expression pattern seen among these genes in the DM2 group (Tab. 3).

The mean value of plasma total [Mg] determined in samples of the DM2-group patients was 0.95 ± 0.07 (SD) mmol.l⁻¹ (n = 56; min. value = 0.78, max. value = 1.12). This concentration of magnesium in blood plasma is considered sufficient (in commonly used clinical tests) and only one patient with a BPM value of below 0.8 could be considered as being slightly magnesium-deficient with regard to plasma magnesium concentration.

The mean value of [Mg²⁺]ᵢ determined in the white-cell fraction of the DM2 blood samples was 0.68 ± 0.2 (SD) mmol.l⁻¹ (n = 56; min. value = 0.35, max. value = 1.10). The concentration of intracellular ionised magnesium varied considerably and was determined to be below 0.5 mmol.l⁻¹ (milli-mol per Liter) in 32.1 % of all DM2 samples.

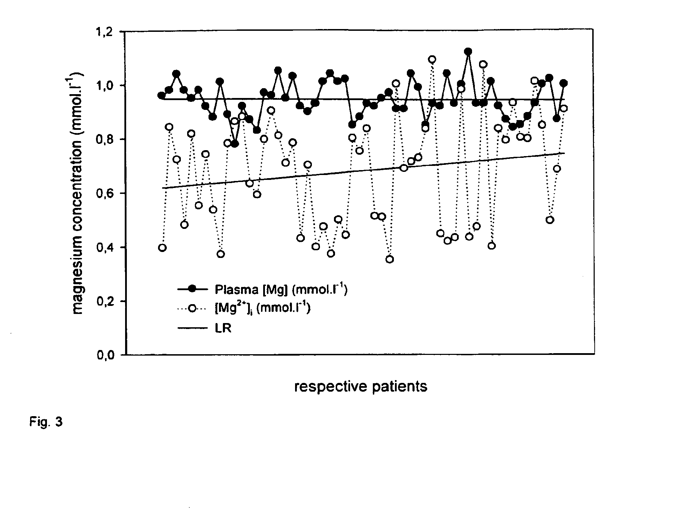

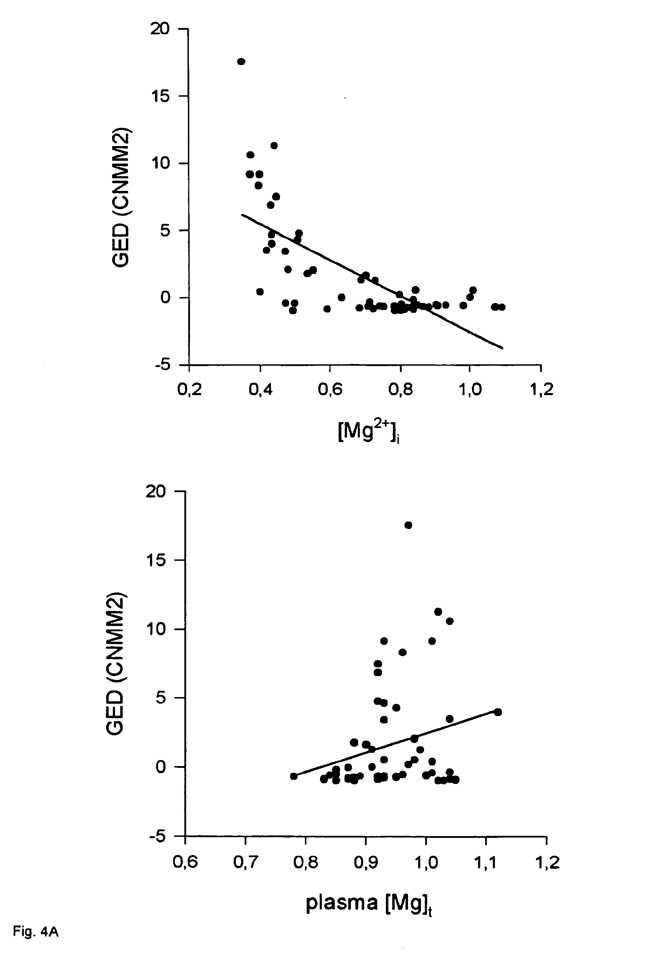

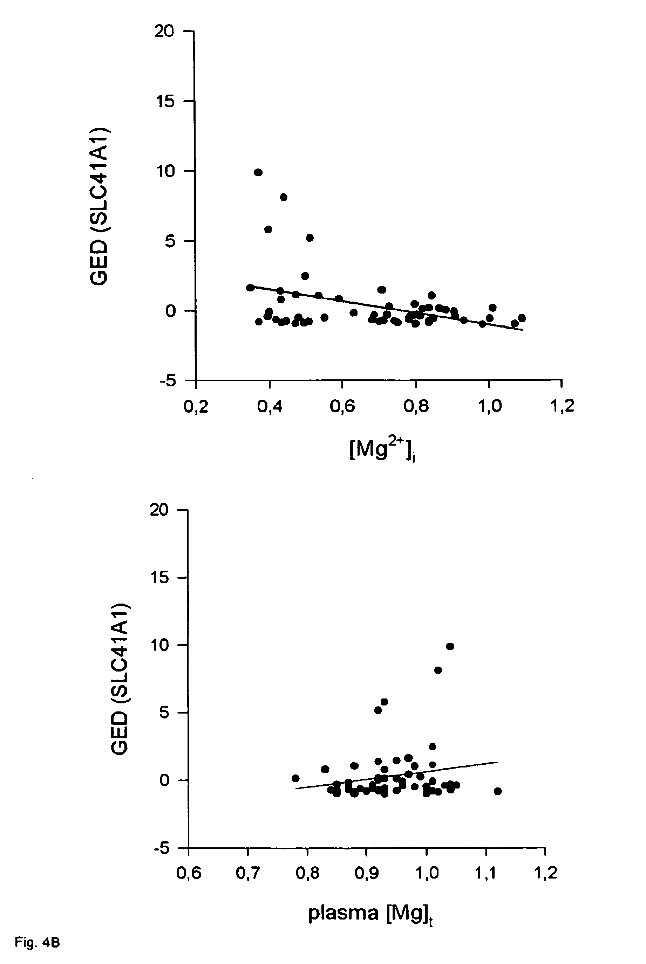

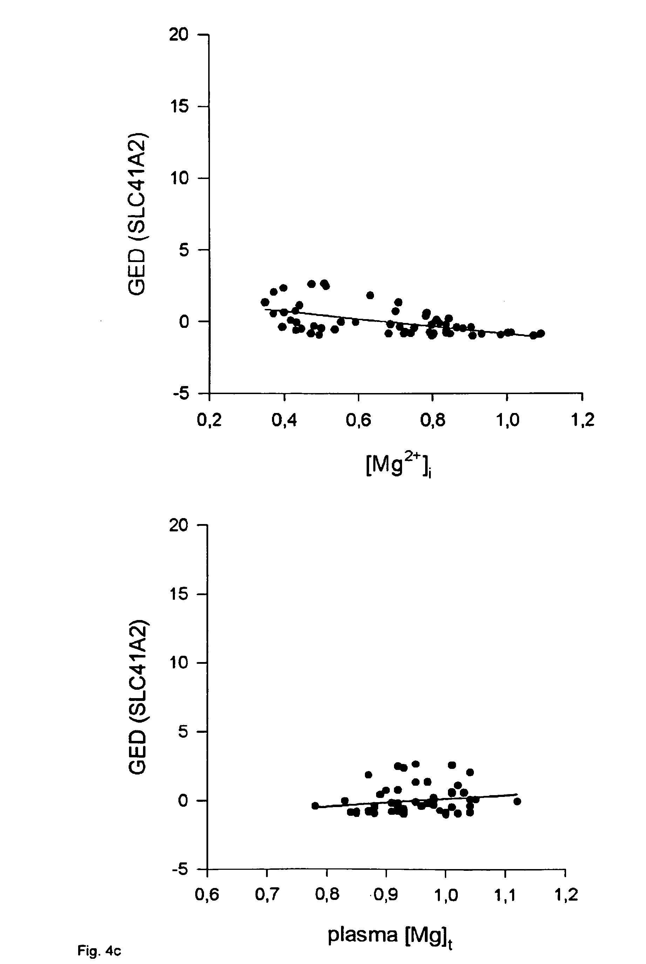

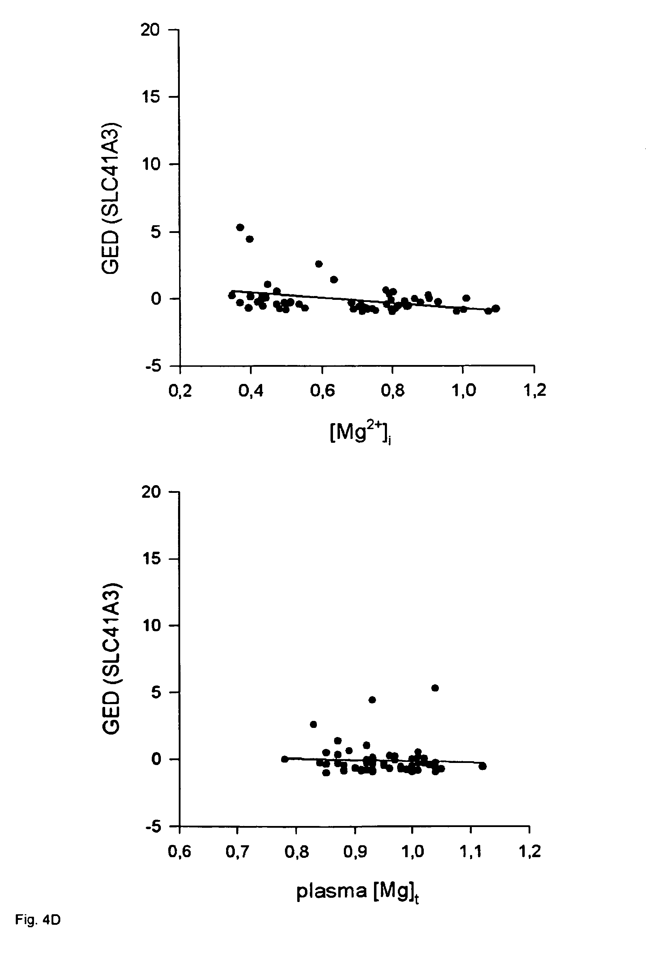

Interestingly, all the patients with an increased expression of the *CNNM2* gene had [Mg²⁺]ᵢ around or below a value of 0.5 mmol.l⁻¹. As evident from Fig. 4, not all DM2 patients with reduced [Mg²⁺]ᵢ to or below of this value also had an increased expression of *SLC41A1, SLC41A2* or *SLC41A3.* Individual values of plasma [Mg]_{T} and [Mg²⁺]ᵢ data acquired for DM2-group patients are plotted in Fig. 3.

The data plotted in Fig. 4 (upper panels) indicate that reduced [Mg²⁺]ᵢ correlates with an increase of expression level in the case of all four genes, but most significantly in the case of gene *CNNM2.* Correlation analyses between the GED values for each of the four genes and the corresponding sets of plasma [Mg]_{T} (Fig. 4; lower panels) revealed no positive or negative relationships between these parameters.

The plots presenting correlations between GED values and the corresponding sets of [Mg²⁺]ᵢ values (Fig. 4.; upper panels) also suggest that, for each of these genes, there are patients with [Mg²⁺]ᵢ < 0.5 mmol.l⁻¹ but with no increase of GED (for CNNM2, 5 patients; in the case of SLC41A1, SLC41A2 and SLC41A3, 10 and more patients). This observation may be indicative of the presence of further, not yet identified, Mg transporter genes having an enhanced expression level correlated with said lowered [Mg²⁺]ᵢ values. Also, it might be possible that not only the plasma [Mg]_{T} but in some instances even the [Mg²⁺]ᵢ values do not necessarily reflect the individual physiology of the respective patients and that their "real" magnesium insufficiency can be determined only when it is reflected in the changed transcription or translation parameters of those genes involved in cellular magnesium homeostasis.

**Table 3**

| | CNNM2 | SLC41A 1 | SLC41A 2 | SLC41A 3 | SLC41A 1 SLC41A 2 | SLC41A 1 SLC41A 3 | SLC41A 2 SLC41A 3 | SLC41A1 SLC41A2 SLC41A3 |
|---|---|---|---|---|---|---|---|---|
| CNNM2 | 11 | 1 | 1 | | 1 | | | 2 |
| | | | | | | | | |

| | CNNM2 | SLC41A 1 | SLC41A 2 | SLC41A 3 | CNNM2 SLC41A 2 | CNNM2 SLC41A 3 | SLC41A 2 SLC41A 3 | CNNM2 SLC41A2 SLC41A3 |
|---|---|---|---|---|---|---|---|---|
| SLC41A 1 | 1 | 1 | | | 1 | | | 2 |
| | | | | | | | | |

| | CNNM2 | SLC41A 1 | SLC41A 2 | SLC41A 3 | CNNM2 SLC41A 1 | CNNM2 SLC41A 3 | SLC41A 1 SLC41A 3 | CNNM2 SLC41A1 SLC41A3 |
|---|---|---|---|---|---|---|---|---|
| SLC41A 2 | 1 | | 1 | | 1 | | | 2 |
| | | | | | | | | |

| | *CNNM2* | *SLC41A 1* | *SLC41A 2* | *SLC41A* 3 | *CNNM2 SLC41A 1* | *CNNM2 SLC41A 2* | *SLC41A 1 SLC41A 2* | *CNNM2 SLC41A1 SLC41A2* |
|---|---|---|---|---|---|---|---|---|
| *SLC41A 3* | | | 1 | | | | | 2 |

### EXAMPLE 2

### Expression levels of CNNM2, SLC41A1, SLC41A2 and SLC41A3 genes in DM2 patients supplemented with Magnesium Diasporal^{®}

A group of 17 DM2 patients who had a normal plasma Mg concentration were selected. All of them over-expressed gene CNNM2, one patient over-expressed genes *CNNM2* and *SLC41A1,* one patient over-expressed genes *CNNM2, SLC41A1* and *SLC41A2* and 2 patients had increased expression levels of all four genes *(CNNM2, SLC41A1, SLC41A2* and *SLC41A3*). These patients were supplemented for a duration of 30 days with Magnesium-Disporal^{®} 300 (magnesium citrate 1830 mg, an effective Mg concentration equivalent to app. 300 mg; Protina Pharm. GmbH), administered once per day. After the second week of the trial, one of the patient developed severe diarrhoea and asthenia (total body weakness). She decided to discontinue her magnesium supplementation as she believed that it was the primary reason for her condition. None of the other patients developed similar symptoms. Sixteen patients who finished the trial gave blood samples and, for 14 of them, the GED values for *CNNM2, SLC41A1, SLC41A2* and *SLC41A3* were determined and compared with corresponding values from the beginning of the trial. Two samples were lost because the filters in the Leukosep^{®} tubes were damaged during the centrifugation step and the leucocyte fraction could no longer be recovered.

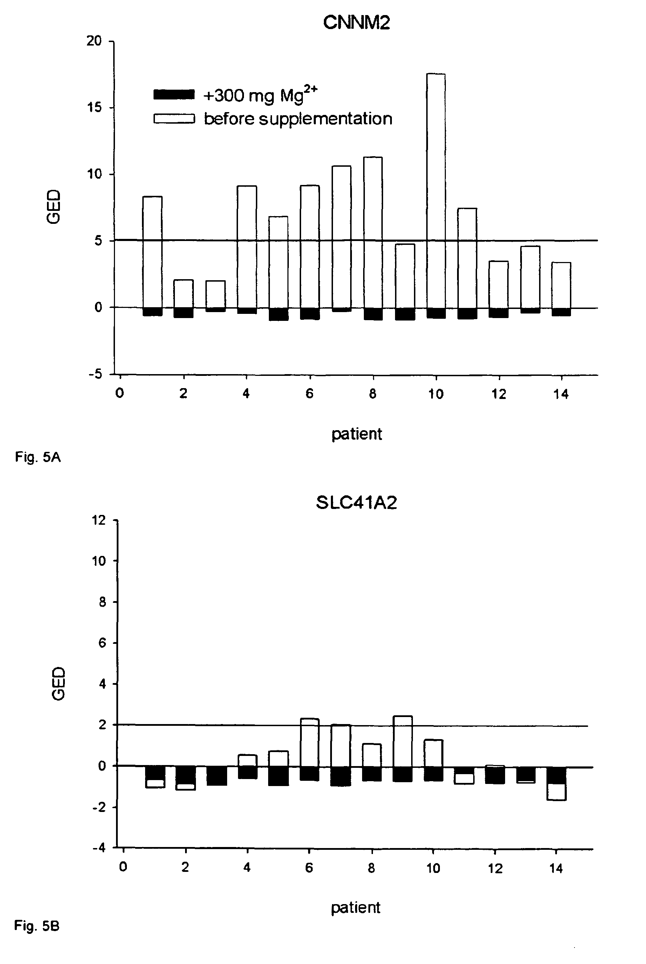

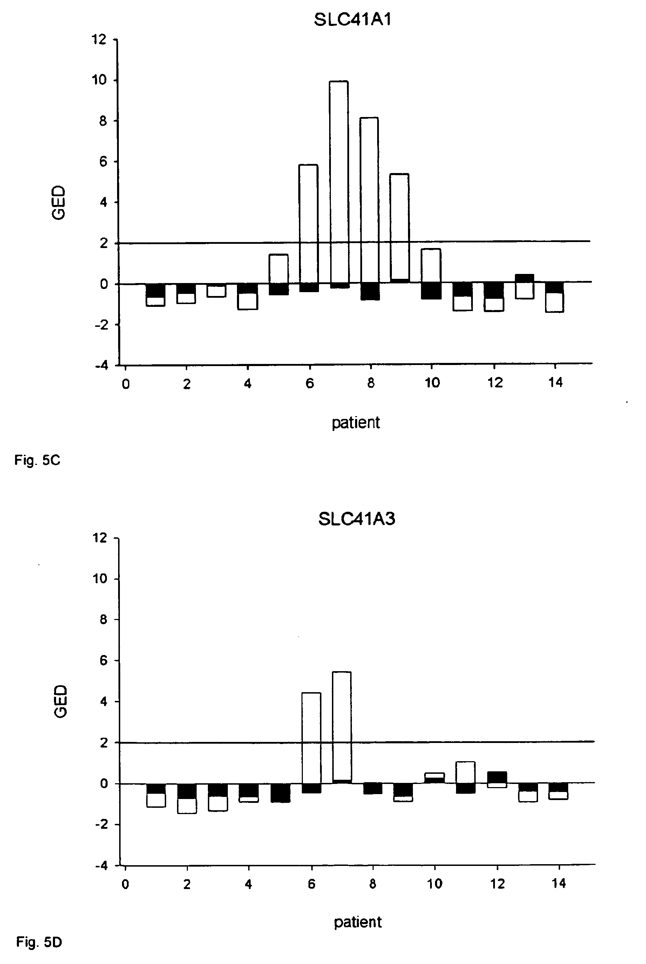

Graphs summarised in Fig. 5 show GED values determined for all four genes in the 14 samples collected after the end of the trial. The expression levels of the studied genes in all samples were normalised compared with the expression levels of these genes in the reference samples. Magnesium supplementation had no effect on the concentration of plasma total magnesium values determined before and after the trial (Fig. 6). [Mg²⁺]ᵢ in the other hand was significantly increased in all analysed samples (n=7). Average [Mg²⁺]ᵢ increased by appr. 30% when it is compared to the average [Mg²⁺]ᵢ value determined before the trial; before trial av. [Mg²⁺]ᵢ 0.43 ± 0.06 (SD) mmol.1⁻¹ (n = 14; min. value = 0.35, max. value = 0.55) and after trial av. [Mg²⁺]ᵢ 0.66 ± 0.09 (SD) mmol⁻¹ (n = 7; min. value = 0.58, max. value = 0.84). These data are in complete agreement with the premise that increase of [Mg²⁺]ᵢ leads to "normal" expression of our genes of interest.

### EXAMPLE 3

### Expression levels of CNNM2, SLC41A1, SLC41A2 and SLC41A3 genes in healthy blood donors supplemented with multi-mineral preparations (control group)

As a positive cross-control for our approach and also for the selection of the reference group of probands, we determined GED values for all four genes in the leucocytes of 17 healthy blood donors. All of them regularly took multi-mineral supplements (magnesium content appr. 100 mg). The mean BPM values determined in the control samples was 0.93 ± 0.07 (SD) mmol.1⁻¹ (n = 17) and ranged from a min. value of 0.79 mmol.l⁻¹ to a max. value of 1.03 mmol.l⁻¹. This range of BPM values is considered clinically normal.

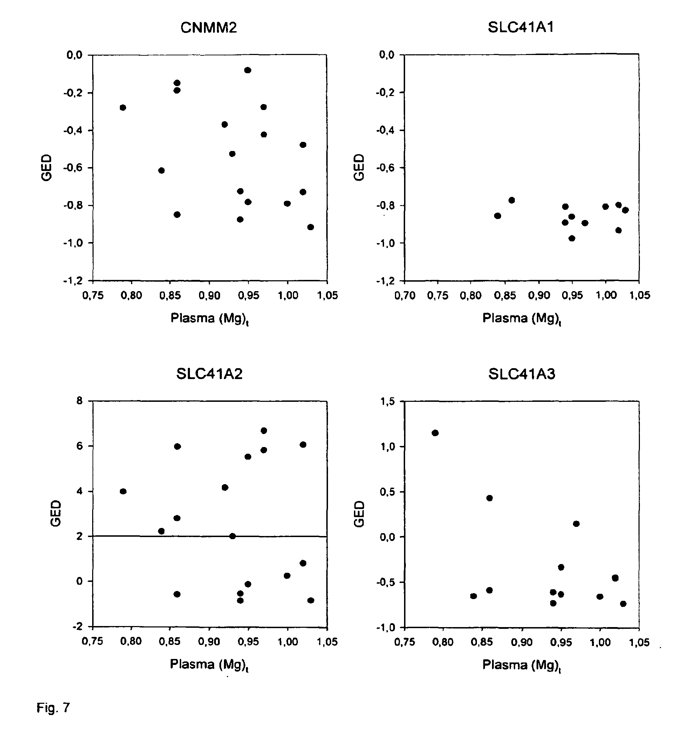

As seen in Fig. 7, the expression levels (GED) of genes *CNNM2, SLC41A1* and *SLC41A3* in control samples were not significantly different from reference samples. However, *SLC41A2* was significantly over-expressed in 58.8% of the control group samples and the max. GED value was three times as high as the *SLC41A2* max. GED value in the DM2 group. This surprising observation might be connected to the multi-mineral supplementation of the probands in control group. Its molecular background can however only be speculated upon at present time. The expression of *SLC41A2* thus responds better in a negative manner to factors other than changes of Mg²⁺ concentration in leucocytes and therefore this gene appears to be less suited for use as a marker gene in the methods disclosed herein.

### Cited references

(1) Wabakken T, Rian E, Kveine M, Aasheim HC. (2003) The human solute carrier SLC41A1 belongs to a novel eukaryotic subfamily with homology to prokaryotic MgtE Mg2+ transporters. Biochem Biophys Res Commun. 306(3):718-24.
(2) Goytain A, Quamme GA. (2005) Functional characterization of human SLC41A1, a Mg2+ transporter with similarity to prokaryotic MgtE Mg2+ transporter. Physiol Genomics. 21(3):337-42.
(3) Kolisek M, Launay P, Beck A, Sponder G, Serafini N, Brenkus M, Froschauer EM, Martens H, Fleig A, Schweigel M. (2008) SLC41A1 is a novel mammalian Mg2+ carrier._J Biol Chem. 283(23):16235-47.
(4) Goytain A, Quamme GA. (2007) Functional characterization of the mouse [corrected] solute carrier, SLC41A2. Biochem Biophys Res Commun. 330(3):701-5. Erratum in: Biochem Biophys Res Commun. 2007 May 11;356(3):822.
(5) Wang CY, Shi JD, Yang P, Kumar PG, Li QZ, Run QG, Su YC, Scott HS, Kao KJ, She JX. (2003) Molecular cloning and characterization of a novel gene family of four ancient conserved domain proteins (ACDP). Gene. 306:37-44.
(6) Goytain A, Quamme GA. (2005) Functional characterization of ACDP2 (ancient conserved domain protein), a divalent metal transporter. Physiol Genomics. 22(3):382-9.
(7) Schmitz C, Perraud AL, Johnson CO, Inabe K, Smith MK, Penner R, Kurosaki T, Fleig A, Scharenberg AM. (2003) Regulation of vertebrate cellular Mg2+ homeostasis by TRPM7. Cell. 114(2):191-200.
(8) Groenestege WM, Hoenderop JG, van den Heuvel L, Knoers N, Bindels RJ. (2006) The epithelial Mg2+ channel transient receptor potential melastatin 6 is regulated by dietary Mg2+ content and estrogens. J Am Soc Nephrol. 17(4):1035-43.
(9) Goytain A, Quamme GA. (2005) Identification and characterization of a novel mammalian Mg2+ transporter with channel-like properties. BMC Genomics. 6(1):48.
(10) Goytain A, Hines RM, El-Husseini A, Quamme GA. (2007) NIPA1(SPG6), the basis for autosomal dominant form of hereditary spastic paraplegia, encodes a functional Mg2+ transporter. J Biol Chem. 282(11):8060-8.

## Claims

1. An *in vitro* method for determining the presence or absence of an intracellular free magnesium deficiency in a human individual, wherein the intracellular free magnesium deficiency is caused by diabetes mellitus type 2, by determining the expression level of at least one cellular Mg²⁺ transporter gene showing a direct correlation between its expression level and the intracellular free magnesium ion concentration [Mg²]ᵢ, the at least one cellular Mg²⁺ transporter gene being *CNNM2,* in a cell sample of said individual and comparing the obtained expression level with a reference value, wherein an increase of the expression level relative to the reference value indicates that the individual suffers from an intracellular free magnesium deficiency.

2. The method according to claim 1, comprising:
a) performing quantitative PCR on the mRNA of a cellular Mg²⁺ transporter gene in a nucleated blood cell sample, preferably a leucocyte sample, of the individual;
b) comparing the value obtained in step a) with a reference value obtained from a sample of a healthy human showing no intracellular free magnesium deficiency,
wherein an increase of the expression level of the cellular Mg²⁺ transporter gene relative to the reference value indicates that the individual suffers from an intracellular free magnesium deficiency.

3. The method according to claim 1 or 2, wherein the intracellular free magnesium deficiency is **characterized by** an intracellular free magnesium ion concentration [Mg²⁺]ᵢ of less than 0.5 mmol/l.

4. The method according to any one of claims 1-3, wherein the increase in the expression level of the cellular Mg²⁺ transporter gene relative to the reference value is at least 2-fold.

## Patentansprüche

1. *In vitro* Verfahren zur Bestimmung des Vorliegens oder Nichtvorliegens eines Mangels an intrazellulärem freiem Magnesium in einem menschlichen Individuum, wobei der Mangel an intrazellulärem freiem Magnesium durch Diabetes mellitus Typ 2 verursacht wird, durch Bestimmen des Expressionslevels von mindestens einem zellulären Mg²⁺-Transportergen, das eine direkte Korrelation zwischen seinem Expressionslevel und der intrazellulären freien Magnesiumionen-Konzentration [Mg²⁺]ᵢ zeigt, wobei das mindestens eine zelluläre Mg²⁺-Transportergen *CNNM2* ist, in einer Zellprobe des besagten Individuums und Vergleichen des bestimmten Expressionslevels mit einem Referenzwert, wobei ein Anstieg des Expressionslevels relativ zu dem Referenzwert bedeutet, dass das Individuum an einem Mangel an intrazellulärem freiem Magnesium leidet.

2. Verfahren nach Anspruch 1, umfassend:
a) Durchführen einer quantitativen PCR mit der mRNA eines zellulären Mg²⁺-Transportergens in einer kernhaltigen Blutzellprobe, vorzugsweise einer Leukozyten-Probe, des Individuums;
b) Vergleichen des in Schritt a) erhaltenen Werts mit einem Referenzwert, der von einer Probe eines gesunden Menschen erhalten wurde, der keinen Mangel an intrazellulärem freiem Magnesium zeigt,
wobei ein Anstieg des Expressionslevels des zellulären Mg²⁺-Transportergens relativ zu dem Referenzwert bedeutet, dass das Individuum an einem Mangel an intrazellulärem freiem Magnesium leidet.

3. Verfahren nach Anspruch 1 oder 2, wobei der Mangel an intrazellulärem freiem Magnesium durch eine intrazelluläre freie Magnesiumionen-Konzentration [Mg²⁺]ᵢ von weniger als 0.5 mmol/l gekennzeichnet ist.

4. Verfahren nach irgendeinem der Ansprüche 1 - 3, wobei der Anstieg des Expressionslevels des Mg²⁺-Transportergens relativ zu dem Referenzwert wenigstens zweifach ist.

## Revendications

1. Procédé *in vitro* de détermination de la présence ou de l'absence d'une insuffisance de magnésium intracellulaire libre dans un individu humain, l'insuffisance de magnésium intracellulaire libre étant causée par le diabète mellitus type II, en déterminant le niveau d'expression d'au moins un gène transporteur Mg²⁺ cellulaire montrant une corrélation directe entre son niveau d'expression et la concentration en ions de magnésium intracellulaire libre [Mg²⁺]_{¡}, ledit au moins un gène transporteur Mg²⁺ cellulaire étant *CNNM2,* dans un échantillon de cellules dudit individu, et en comparant le niveau d'expression obtenu à une valeur de référence, une augmentation du niveau d'expression par rapport à la valeur de référence indiquant que ledit individu souffre d'une insuffisance de magnésium intracellulaire libre.

2. Procédé selon la revendication 1, comprenant:
a) mettre en oeuvre une PCR quantitative avec l'ARNm d'un gène transporteur Mg²⁺ cellulaire dans un échantillon de cellules sanguines nucléées, de préférence un échantillon de leucocytes, de l'individu ;
b) comparer la valeur obtenue à l'étape a) à une valeur de référence obtenue à partir d'un échantillon d'un être humain sain ne montrant pas d'insuffisance de magnésium intracellulaire libre,
une augmentation du niveau d'expression du gène transporteur Mg²⁺ cellulaire par rapport à la valeur de référence indiquant que ledit individu souffre d'une insuffisance de magnésium intracellulaire libre.

3. Procédé selon la revendication 1 ou 2, dans lequel l'insuffisance de magnésium intracellulaire libre est **caractérisée par** une concentration en ions de magnésium intracellulaire libre [Mg²⁺]ᵢ inférieure à 0.5 mmol/l.

4. Procédé selon l'une quelconque des revendications 1 à 3, dans lequel l'augmentation du niveau d'expression du gène transporteur Mg²⁺ par rapport à la valeur de référence est au moins le double.
